# EUROPEAN PATENT SPECIFICATION

(11) **EP 0 525 655 B1**
(45) Date of publication and mention of the grant of the patent: **20.12.1995**
(21) Application number: 92112638.9
(22) Date of filing: 23.07.1992
(51) Int. Cl.: A61K 9/127, A61K 9/00

(54) **Formulations useful for vaginal dryness treatment**
Formulierungen zur Behandlung der vaginalen Trockenheit
Formulations pour le traitement de la sécheresse vaginale

(30) Priority: 02.08.1991 IT MI912181
(43) Date of publication of application: 03.02.1993
(73) Proprietor: POLI INDUSTRIA CHIMICA S.p.A., 20141 Milano (IT)
(72) Inventor: Poli, Stefano, I-20089 Quinto De' Stampi-Rozzano (Mil.) (IT); Moro, Luigi, I-20089 Quinto De' Stampi-Rozzano (Mil.) (IT); Busetti, Cesare, I-20089 Quinto De' Stampi-Rozzano (Mil.) (IT); Merico, Roberto, I-20089 Quinto De' Stampi-Rozzano (Mil.) (IT)
(74) Representative: Bianchetti, Giuseppe

(56) References cited:
- EP-A- 0 065 385
- EP-A- 0 450 352
- WO-A-91/06283
- FR-A- 2 660 192
- US-A- 3 957 971
- SÖFW: SEIFEN, ÖLE, FETTE, WACHSE vol. 117, no. 10, 26 June 1991, AUGSBURG (DE) pages 372 - 378 , XP228115 J. R DING ET AL. 'Beeinflussung der Hautfeuchtigkeit durch Liposomen'

## Description

The present invention relates to liposome formulations suitable for vaginal administration.

Liposomes have known for a long time as potential carriers of pharmacologically active compounds and their use in therapy is the object of about 10,000 publications besides many patents.

Liposomes were described for the first time about 25 years ago, when Bangham discovered that some molecules having particular characteristics, such as phospholipids, interact with water to spontaneously form little hollow spheres full of water.

Liposomes are then spheric vesicles which are delimited by double layered phospholipidic membranes, which are alternated with discrete and isolated aqueous spaces.

Several kind of structures can be distinguished, such as unilamellar vesicles, wherein the liposome is formed by a single double-layered membrane, or multilamellar vesicles, wherein the liposome is formed by several concentric layers of double-layered membranes, each being separated from the other by a water layer. Liposomes may size from a ten of nanometers to some micrometers in diameter.

Over the physico-chemical stability of the resulting structures, the liposome vesicle composition, whose components widely occur in biologic membranes, also affects and defines their carrier capability.

Liposomes have already been used in cosmetic, due to their cutaneous hydrating capacity. However, no liposome formulations for gynaecologic use are known.

US 3,957,971 discloses a liposome composition capable of moisturizing keratinous matter. EP 0,450,352 discloses liposome formulations as drug carriers for immunomodulating drug for topical and aerosol administrations. Röding et al (SÖFW, vol. 117, n. 10 (1991), 372-378) describe the use of liposomes for the control of skin humidity and the stabilization of skin care oils and lipophilic substances with liposomes.

The present invention provides the use of liposomes as hydrating agents for the treatment of vaginal dryness conditions.

Normally, muco-cervical gland secretion covers vaginal epithelium with an aqueous film, whose volume, pH and composition changes along with age and menstrual cycle phase.

A correct vaginal hydration and lubrication play an extremely important role in the sexual life of the couple, in fact the reduction or lack of lubricating secretion combined with a reduction of the vaginal tissue elasticity and trophism, can involve a symptomatology having variable gravity (Dyspareunia), which is however always feeled by the woman with discomfort.

The above situation may occur in the following conditions:
1. Menopause: reduced vaginal mucosae trophism, combined with reduced production of secretions from lesser vestibular glands;
2. Puerperium and lactation: episiorraphy algesiogenic exitus, menopause-like characteristics of vaginal mucosa in nursing mothers;
3. Severe acute vaginitis and chronical forms: reduced vaginal mucosae elasticity and reduced capacity to bear the "traumatisms" of a sexual intercourse;
4. Vaginismus: the difficulty in performing the sexual intercourse, more often due to psychologic reasons.

From literature data a high percentage of women suffering from reduced vaginal hydration efficiency is noticed on the other hand, the increased mean life index, together with the way of living evolution of the couple, requires the use of products having hydrating and lubricating activity on the vagina.

The formulations of the invention allow a particularly effective and convenient treatment of the above mentioned pathological and/or dismetabolic conditions because of the increased water supply to the deep mucosa layers due to the high affinity of liposomes with biologic membranes.

Table 1 shows the results of the treatment with 5 g/day of liposomes on 12 post-menopausal patients suffering from vaginal mucosa atrophy and dryness.

In the table, the symptomatology modifications at vulvo-vaginal level are reported.

**TABLE 1**

| Clinical symptomatology | Basal | 1^{st} day | 2^{nd} day | 7^{th} day |
|---|---|---|---|---|
| Erythema | 2 | 2 | 2 | 1 |
| Itching | 3 | 2 | 1 | 0 |
| Burning sensation | 3 | 1 | 0 | 0 |
| Dryness | 3 | 0 | 0 | 0 |
| Dyspareunia | 3 | 1 | 1 | 0 |
| [3 = marked 2 = medium 1 = light 0 = absent] | | | | |

1 to 5 g/day of vaginally administered liposomes proved to be active also in 15 puerperae with episiorraphy and/or vaginal mucosae atrophy exitus, as reported in Table 2.

**TABLE 2**

| Clinical symptomatology | Basal | End of 1^{st} week | End of 2^{nd} week |
|---|---|---|---|
| Dryness | 3 | 0 | 0 |
| Pelvic algiae | 3 | 1 | 0 |
| Dyspareunia | 3 | 1 | 1 |
| Dysuria | 3 | 1 | 0 |
| [3 = marked 2 = medium 1 = light 0 = absent) | | | |

The formulations of the invention consist of liposome suspensions prepared by dispersing one or more phospholipidic, sphyngolipidic, glycolipidic or proteolipidic substances together with bioadhesive polymers in a solvent or in an aqueous solvent mixture. The above substances perform a plastic function of carrying structure of the liposome carrier, by virtue of their natural capacity to form a bilayer when hydrated.

Agents capable of giving the liposome bioadhesive characteristics are, for example, biopolymers (e.e. hyaluronic acid or polygalacturonic acid or collagen) or chemically derived biopolymers (e.g. cellulose or acrylic derivatives).

The liposome suspension according to the invention can further contain one or more of the following compounds:
1. Transition temperature modulating agents, such as cholesterol or esters thereof typical of each phospholipid;
2. Charge bearing substances which, at the pH of the preparation, are partially or totally dissociated, therefore act as charge bearing agents capable of giving the liposome a net positive or negative surface charge. Examples of such substances are diacetyl phosphate, phosphatidic acid, phosphatidyl serine, phosphatidylethanolamine, stearylamine, cardiolipin;
3. Antioxidant agents capable of inhibiting double bond peroxidation in fat acids, which are contained in lipidic substances. Examples of such substances are tocopherols, the esters thereof, butylhydroxyanisole, butylhydroxytoluene, β-carotene;
4. Chelating agents, capable of complexing by coordination bonds multivalent metal ions which can act as aggregation nuclei of liposome structures, or as catalysts for oxydative-type demolition reactions;
5. Hydrating agents, capable of enhancing the liposome system specific activity, such as, for example, alcohols, polyhydroxylated glycols, mucopolysaccharides, and the like;
6. Agents capable of giving the liposome carrier thermoviscosity characteristics and facilitating adhesion to the mucosa, such as for example, polyoxyethylene, polyoxypropylene polimer, known under the trade name Lutrol F127®;
7. Mild decongesting agents such as dry, soft, aqueous, hydroalcoholic, hydroglycolic extracts of wild chamomile, malva, althea hamamelis and the like;
8. Wound healing agents, such as, for example, Triticum vulgare, Centella asiatica extracts, hyaluronic acid or derivatives thereof and the like;
9. Disinfecting or antibacterial agents, such as parahydroxybenzoic acid ethers, benzoic acid and the esters thereof, sorbic acid and the salts thereof, dehydroacetic acid and the salts thereof;
10. Optional excipients, such as perfumes and essences, dyes, and the like.

The solvent phase exclusively or mainly consists of water, small percentages of ethanol or other compatible solvents being optionally present (generally lower than about 15%).

A particularly preferred embodiment of the invention consists in using multilamellar liposomes, which can be obtained, for example, according to the methods described in Remington's Pharmaceutical Sciences Handbook, Mack Publ. Co., New York, U.S.A., 18th edition, page 1691.

Some typical parameters of liposome formulations, such as liposome internal volume, trapping efficacy may vary within rather broad limits, which are anyway easily determinable by those skilled in the art.

Internal volume means the water trapped in the liposome structure per lipid unit (»l/mmole or »l/mg of total lipids), whereas trapping efficacy usually means the ratio between trapped marker percentage and the free marker one. Some typical internal volume values are summarized in the following table:

| Liposome type | Internal volume |
|---|---|
| SUV | < 0.5 |
| MLV | > 4 |
| REV | > 10 |

Said values strongly depend on some parameters, among which the composition to be used, lipid concentration therein and the preparation method.

The use of negatively charged lipids, such as phosphatidylserine, phosphatidylinositol, phosphatidylglycerol and the like, or the use of positively charged surfactants, such as stearylamine, tend to increase water trapping efficacy by increasing interlamellar distance between subsequent bilayers of the multilamellar structures.

It is well-known that trapping efficacy is widely affected by the preparation method.

Several methods are described in literature, which, by working with different techniques, can lead to remarkably different values for this parameter (see for instance M. Riaz, N. Weiner, F. Martin in H. Lieberman, N. Rieger, G. Banker Eds.: Pharmaceutical Dosage Forms - Disperse systems, vol. 2).

Sometimes, increasing the already high liposome bioadhesive power, by using suitable natural or synthetic polymers, such as alginates, collagen, cellulose or acrylic derivatives (Carbomer®, Polycarbophil®) could be convenient.

Although the liposomic suspensions of the present invention are already particularly efficient as water carrier by themselves, sometimes it could be convenient to add other conventional active ingredients having adjuvant and hydrating activity, such as alcohols or polyhydroxylated glycols, mucopolysaccharides, lactic acid or the salts thereof, 2-pyrrolidon-5-carboxylic acid or the salts thereof, vegetal or mineral oils, silicone derivatives.

The preparation of the liposome suspensions of the invention is carried out according to conventional techniques, for example, by dispersing an alcoholic solution of lipidic material into an aqueous solution which can optionally contain preserving, chelating, hydrating agents and the like. After eliminating the solvent by evaporation to achieve residual values preferably lower than 5%, a liposome suspension is obtained.

Said suspensions can directly be used for vaginal applications by means of suitable supplying devices.

Alternatively, the liposome suspensions can be formulated, according to conventional techniques, for example, as described in Remington's Pharmaceutical Sciences Handbook, Mack Publ. Co., New York, U.S.A. 18^{th} edition, in appropriate forms of vaginal administration, such as lavages, gels, emulsions, suspensions, structured vehicles and the like. The formulations of the inventions can be applied one or more times a day, in doses ranging from 0.1 to more than 10 g per application, according to patient conditions.

The following examples further illustrate the invention.

### EXAMPLE 1

7.5 g of lecithin, 1.5 g of cholesterol, 0.9 g of dicetyl phosphate and 0.04 g of tocopherol acetate were dissolved into the minimum amount of ethyl alcohol.

This solution was dispersed under stirring into 200 ml of an aqueous solution containing 0.1% of sodium acetate, 0.15% of sodium parabenates and q.s. of lactic acid.

After evaporating off the solvent until a residual amount lower than 3% w/v, pH of the liposome suspension was 4.7 and the net surface charge was negative.

The suspension was partitioned into monodose containers provided with cannula for vaginal applications and resulted suitable for the treatment of the above mentioned situations of vaginal dryness.

### EXAMPLE 2

100 ml of liposomal suspension were prepared as described in example 1 and then gelifyied by adding 1.5% w/v of sodium alginate.

A well spreadable gel was obtained, whose consistency was suitable for vaginal administration, by means of a specific applicator, to treat vaginal dryness.

### EXAMPLE 3

A liposome suspension, having pH 4.8 and no specific net charge, was prepared as described in example 1 with the same amounts of auxiliary substances, but eliminating dicetyl phosphate.

A liposome suspension, usable as vaginal lavage in the above mentioned dryness situations, was obtained.

### EXAMPLE 4

1% w/v of hydroxypropylmethylcellulose and 1% of collagen were added to 100 ml of liposome suspension obtained according to example 3. A well-spreadable gel having appropriate consistency for the above mentioned applications was obtained.

### EXAMPLE 5

A liposome suspension, having positive net surface charge, was prepared according to example 1 with the same amounts of auxiliary substances, but using an analogous amount of stearylamine instead of dicetyl phosphate. The so-obtained liposome suspension was suitable to be used as vaginal lavage in the above-mentioned situations of vaginal dryness.

### EXAMPLE 6

1% w/v of hydroxypropylmethylcellulose and 1% of collagen were added to 100 ml of liposome suspension obtained according to example 5. A well-spreadable gel having appropriate consistency for the above mentioned applications was obtained.

### EXAMPLE 7

50 g of lecithin, 12 g of cholesterol, 1 g of antioxidant agent, known under the trade name Anoxid SBN® (in Italy Formenti) were added to about 100 ml of ethyl alcohol. Separately, 950 ml of aqueous solution, containing 0.25% w/v of Tricetol, 3% of propylene glycol and 1.1% of chamomile hydroglycolic extract, were prepared.

The alcoholic solution was added to the aqueous solution under vigorous stirring and alcohol was evaporated with gentle heating until a residual amount not higher than 3% w/v.

The so obtained liposome suspension was gelified by adding a cellulose derivative, known under the trade name Natrosol HR®, until an appropriate consistency for vaginal administration was achieved.

After suitable packaging, the product resulted fit for the above mentioned situations.

### EXAMPLE 8

70 g of lecithin, 22 g of cholesterol, 3 g of stearylamine and 1.5 g of tocopherol acetate were dissolved into the minimum amount of ethyl alcohol.

The solution was dispersed under vigorous stirring into 900 ml of an aqueous solution containing 0.1% of sodium edetate and 0.2% of sodium methylparahydroxybenzoate.

The so obtained suspension was put under reduced pressure as to remove part of the alcoholic solvent; then 100 ml of a solution containing 1% w/v of malva hydroglycolic extract and 2% w/v of a hydrating mixture, known under the trade name Lactil® (available in Italy from TEGO), were added.

### EXAMPLE 9

850 g of 0.1% w/v sodium edetate and 0.2% w/v sodium methylparahydroxybenzoate aqueous solution were added to 150 g of a pre-liposomed gel solution, known under the trade name Natipide II® (available in Italy from Rhône Poulenc - Italia).

pH was adjusted with lactic acid q.s. to 5. The suspension was partitioned into monodose containers provided with cannula for the treatment of vaginal dryness.

### EXAMPLE 10

1% of carboxyvinyl polymer, peviously preswollen in water overnight, was added to part of the liposome solution of example 9. A translucent gel, having the appropriate viscosity to be used with a special vaginal applicator in vaginal dryness situations, was obtained.

### EXAMPLE 11

20 g of lecithin, 6.5 g of cholesterol, 0.15 g of tocopherol acetate and 0.5 g coconut fat acid diethanolamine were dissolved into about 100 ml of ethyl alcohol.

The solution was dispersed under whirling stirring into 900 ml of 0.1% sodium edetate, 0.2% sodium benzoate, 0.2% Tricetol, 1.1% w/v chamomile hydroglycolic extract and 10% w/v propylene glycol aqueous solution.

The so obtained solution was put under reduced pressure as to remove part of the alcoholic solvent and pH was adjusted with lactic acid q.s. to 5.

A suspension which, appropriately packaged, was capable of generating a soft foam suited for using, with a special applicator, in vaginal dryness situations, was obtained.

### EXAMPLE 12

75 g of lecithin, 15 g of cholesterol, 5 g of dicetyl phosphate and 0.4 g of tocopherol acetate were dissolved into 100 ml of ethyl alcohol.

Separately, 900 ml of 5% propylene glycol, 5% of hyaluronic acid sodium salt aqueous solution were prepared. The alcoholic solution was added to the aqueous solution under vigorous stirring and the alcohol was let evaporate by gently heating under vacuum. A well spreadable gel was obtained, whose consistency was suitable for vaginal administration, by means of a specific applicator, to treat vaginal dryness.

## Claims

1. Vaginally administrable liposome formulations suitable for the hydrating and/or lubricating vaginal treatment consisting of one or more phospholipidic, sphyngolipidic, glycolipidic or proteolipidic substances; bioadhesive polymers and a continuous aqueous solvent phase.

2. Formulations according to claim 1, further containing one or more of the following agents:
- cholesterol and the esters thereof;
- ionizable group bearing agents;
- antioxidant agents;
- chelating agents;
- hydrating agents;
- agents capable of giving the liposomal structure thermoviscosity characteristics;
- decongesting agents;
- disinfecting and/or antibacterial agents;
- excipients such as perfumes, essences, dyes.

3. Liposome formulations according to claims 1 or 2 characterized in that ethanol or other compatible solvents are present.

4. Liposome formulations according to claim 1 characterized in that Carbopol 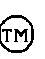 , cellulose or acrylic derivatives, alginates, collagen or hyaluronic acid and derivatives thereof are the polymers.

5. Liposome formulations according to any one of the preceding claims, characterized in that said formulations contain polymers capable of giving the suspension thermoviscosity characteristics.

6. Liposome formulations according to claim 5, characterized in that Lutrol F127 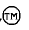 or Pluronic F127 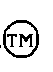 is the polymer.

7. Liposome formulations according to any one of the preceding claims, characterized in that the suspension contains hydrating agents selected from the group consisting of alcohols, polyhydroxylated glycols, mucopolysaccharides, lactic acid or the salts thereof, 2-pyrrolidon-5-carboxylic acid of the salts thereof, vegetal or mineral oils, silicone derivatives.

8. Liposome formulations according to any one of the preceding claims, in the form of lavage, gel, emulsion, suspension, structured vehicle.

9. The use of liposomes as hydrating agents for the manufacturing of hydrating formulations according to anyone of claims 1-8 suited for the hydrating and/or lubricating vaginal treatment.

## Patentansprüche

1. Vaginal verabreichbare Liposomenformulierungen, geeignet für die hydratisierende und/oder die Gleitfähigkeit erhöhende Vaginalbehandlung, bestehend aus einer oder mehreren Phospholipid-, Sphingolipid-, Glycolipid oder Proteolipidsubstanzen, bioadhäsiven Polymeren und einer kontinuierlichen wäßrigen Lösungsmittelphase.

2. Formulierungen nach Anspruch 1, dadurch **gekennzeichnet,** daß sie weiterhin eines oder mehrere der folgenden Mittel enthalten:
- Cholesterin und die Ester davon;
- ionisierbare Gruppen tragende Mittel;
- Antioxidantien;
- chelatbildende Mittel;
- hydratisierende Mittel;
- Mittel mit der Fähigkeit, der Liposomenstruktur Thermoviskositätseigenschaften zu verleihen;
- Decongestionsmittel;
- Desinfektionsmittel und/oder antibakterielle Mittel;
- Excipientien, wie Duftstoffe, Essenzen, Farbstoffe.

3. Liposomenformulierungen nach den Ansprüchen 1 oder 2, dadurch **gekennzeichnet,** daß Ethanol oder andere verträgliche Lösungsmittel vorhanden sind.

4. Liposomenformulierungen nach Anspruch 1, dadurch **gekennzeichnet,** daß die Polymere Carbopol 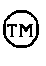 , Cellulose- oder Acrylsäurederivate, Alginate, Collagen- oder Hyaluronsäure und Derivate davon sind.

5. Liposomenformulierungen nach einem der vorausgehenden Ansprüche, dadurch **gekennzeichnet,** daß die Formulierungen Polymere mit der Fähigkeit, der Suspension Thermoviskositätseigenschaften zu verleihen, enthalten.

6. Liposomenformulierungen nach Anspruch 5, dadurch **gekennzeichnet,** daß das Polymere Lutrol F127 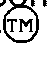 oder Pluronic F127 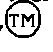 ist.

7. Liposomenformulierungen nach einem der vorausgehenden Ansprüche, dadurch **gekennzeichnet,** daß die Suspension hydratisierende Mittel, ausgewählt aus der Gruppe bestehend aus Alkoholen, polyhydroxylierten Glykolen, Mucopolysacchariden, Milchsäure oder Salzen davon, 2-Pyrrolidon-5-carbonsäure oder Salzen davon, pflanzlichen oder mineralischen Ölen, Siliconderivaten, enthält.

8. Liposomenformulierungen nach einem der vorausgehenden Ansprüche in Form einer Waschlösung, eines Gels, einer Emulsion, einer Suspension, eines strukturierten Trägers.

9. Verwendung von Liposomen als hydratisierende Mittel zur Herstellung von hydratisierenden Formulierungen nach einem der Ansprüche 1-8 mit der Eignung zur hydratisierenden und/oder die Gleitfähigkeit erhöhenden Vaginalbehandlung.

## Revendications

1. Formulations de liposomes administrables par voie vaginale utilisables pour les traitements vaginaux hydratants et/ou lubrifiants consistant en une ou plusieurs substances phospho-lipidiques, sphyngolipidiques, glycolipidiques ou protéolipidiques; en polymères bioadhésifs et en une phase de solvant aqueux continue.

2. Formulations suivant la revendication 1, contenant de plus, un ou plusieurs des agents suivants :
- cholestérol et ses esters;
- agents portant un groupe ionisable;
- agents antioxydants;
- agents chélatants;
- agents hydratants;
- agents capables de donner des caractéristique de thermoviscosité à la structure de liposome;
- agents décongestionnants;
- agents désinfectants et/ou antibactériens;
- excipients tels que parfums, essences, colorants.

3. Formulations de liposomes suivant les revendications 1 et 2, caractérisées en ce que de l'éthanol ou d'autres solvants compatibles sont présents.

4. Formulations de liposomes suivant la revendication 1, caractérisées en ce que du Carbopol™, des dérivés cellulosiques ou acryliques, des alginates, du collagène ou de l'acide hyaluronique et leurs dérivés, sont les polymères.

5. Formulations de liposomes suivant l'une quelconque des revendications précédentes, caractérisées en ce que ces formulations contiennent des polymères capables de donner des caractéristiques de thermoviscosité à la suspension.

6. Formulations de liposomes suivant la revendication 5, caractérisées en ce que le Lutrol F127™ ou le Pluronic F127™, est le polymère.

7. Formulations de liposomes suivant l'une quelconque des revendications précédentes, caractérisées en ce que la suspension contient des agents hydratants choisis dans le groupe consistant en alcools, glycols polyhydroxylés, mucopolysaccharides, acide lactique ou ses sels, acide 2-pyrrolidon-5-carboxylique ou ses sels, huiles végétales ou minérales et dérivés silicones.

8. Formulations de liposomes suivant l'une quelconque des revendications précédentes, sous forme de lavage, gel, émulsion, suspension, véhicule structuré.

9. Utilisation de liposomes comme agents hydratants pour la préparation de formulations hydratantes selon l'une quelconque des revendications 1 à 8, adaptées au traitement vaginal hydratant et/ou lubrifiant.
